# DEMANDE DE BREVET EUROPEEN

(11) **EP 0 549 388 A1**
(43) Date de publication de la demande: **30.06.1993**
(21) Numéro de dépôt: 92403267.5
(22) Date de dépôt: 03.12.1992
(51) Int. Cl.: C12Q 1/68

(54) **Procédé de sélection d'au moins un crible de mutations, son application à un procédé d'identification rapide d'alleles de systèmes polymorphes et dispositif pour sa mise en oeuvre**

(30) Priorité: 04.12.1991 FR 9114996
(71) Demandeur: BERTIN & CIE, F-78373 Plaisir Cédex (FR)
(72) Inventeur: Cohen, Nadine, F-75019 Paris (FR); Bougueleret, Lydie, F-75015 Paris (FR); Cohen, Daniel, F-94160 saint Mande (FR); Dausset, Jean, F-75007 Paris (FR)
(74) Mandataire: Orès, Bernard

(57) **Abrégé**

Procédés de sélection d'au moins un crible de mutations à partir d'un ensemble de séquences allèliques d'un gène polymorphe et d'identification rapide d'allèles de gènes polymorphes, sondes nucléotidiques obtenues à partir desdits cribles de mutations, notamment constitués en banque de données et dispositif pour la mise en oeuvre desdits procédés. Le procédé pour l'identification d'allèles comprend : (a) la sélection de tout ou partie d'une séquence *consensus* connue dudit gène polymorphe ; (b) la création d'une matrice des mutations des séquences correspondantes d'allèles connus (c) l'identification des séquences indiscernables par comparaison deux à deux (allèles ayant le même profil de mutations dans la séquence sélectionnée en (a)) et l'exclusion d'un des membres desdits couples ; (e) l'identification et le dénombrement des mutations obligatoires ou mutations dites marqueurs d'allèles, c'est-à-dire celles qui sont nécessaires et suffisantes pour la distinction de deux allèles par ailleurs identiques (ensemble O des mutations obligatoires) ; et (f) l'obtention dudit/desdits cribles minimaux de mutations, comprenant au moins les mutations obligatoires de l'étape (e) ; puis (g) le choix, parmi les cribles sélectionnés à l'étape (f) dudit procédé de sélection de cribles de mutations, du crible de mutations le plus adapté à la réalisation de sondes oligonucléotidiques aptes à être utilisées pour la différenciation de tous les allèles ; (h) une hybridation appropriée d'un allèle X à identifier avec les sondes oligonucléotidiques sélectionnées à partir du/des cribles de mutations établis au cours des étapes (a) à (g) ; et (i) identification de l'allèle X par détection dudit/desdits hybrides, éventuellement formés au cours de l'étape (h).

## Description

La présente invention est relative à un procédé de sélection d'au moins un crible de mutations à partir d'un ensemble de séquences allèliques d'un gène polymorphe, à un procédé d'identification rapide de variations allèliques (allèles ou séquences allèliques) des séquences de gènes polymorphes, à des sondes nucléotidiques obtenues à partir desdits cribles de mutations, notamment constitués en banque de données ainsi qu'à un dispositif pour la mise en oeuvre desdits procédés.

La présente invention est également relative à un kit pour l'identification des allèles de gènes polymorphes.

A l'heure actuelle, il est très difficile et très fastidieux d'identifier les différents allèles d'un même gène, se distinguant par mutation d'au moins une base dans leur séquence nucléotidique, notamment dans le cas de systèmes naturellement polyallèliques, tel que le système majeur d'histocompatibilité (HLA) dont les gènes peuvent se présenter sous de très nombreuses formes allèliques, ainsi que dans toute autre forme de polymorphisme, notamment ceux dûs à des mutations somatiques comme celles des immunoglobulines et des récepteurs des cellules T ou encore ceux rencontrés dans des systèmes équivalents à un système polyallèlique, plus particulièrement observés dans certaines maladies génétiques à mutations multiples telles que la mucoviscidose ou la dystrophie musculaire de Duchenne.

Les gènes du complexe majeur d'histocompatibilité (complexe HLA), par exemple, sont étroitement liés sur le bras court du chromosome 6 et s'étendent sur environ 5 000 kB ; ils codent pour trois types de protéines, les protéines de classe I, II et III ; une caractéristique majeure du système HLA est son vaste polymorphisme.

Le polymorphisme de ce système résulte du nombre de gènes et du nombre des différents allèles possibles pour chacun de ces gènes, le polymorphisme étant encore accru si l'on tient compte du fait qu'un individu peut avoir reçu le même allèle de ses deux parents (état homozygote) ou peut avoir reçu deux allèles différents (état hétérozygote).

De plus, si l'on considère que pour le complexe HLA, il peut exister de 10 à 100 allèles par gène et qu'on a actuellement caractérisé 15-20 gènes codant pour les protéines du complexe HLA, il est quasiment impossible d'effectuer un typage (ou identification) complet de ce complexe avec les méthodes actuellement disponibles, alors que ce dernier peut se révéler crucial, notamment en transplantation.

En effet, le typage des différents systèmes polymorphes peut être réalisé, actuellement, soit par des méthodes immunochimiques, soit par des techniques d'hybridation ADN/ADN ; toutefois ces techniques ont l'inconvénient :
. de ne pas être assez discriminatives, et donc de ne pas permettre la différenciation d'allèles de structures très proches et
. de nécessiter l'utilisation d'un nombre élevé de sondes oligonucléotidiques (par exemple : 50-60 sondes environ dans le cas du gène DRβ du système HLA (voir notamment la nomenclature des facteurs du système HLA, publiée en 1990 dans Immunogenetics, 31, 131-140), qui comporte 56 allèles), et ce, dans la mesure où dans les procédés de typage classique par biologie moléculaire de l'art antérieur, il est effectivement nécessaire de prévoir de l'ordre d'une sonde par allèle pour pouvoir interpréter les résultats.

Or, cette identification est souvent nécessaire soit pour des raisons préventives, soit pour des raisons curatives (thérapie, chirurgie, greffes notamment) ; plus particulièrement, dans le cas du complexe HLA, la maîtrise d'un système de typage fiable, est rendue nécessaire dans un but préventif par l'existence d'une corrélation entre la susceptibilité à certaines maladies et la fréquence de certains allèles HLA ; et dans un but curatif par la nécessité d'avoir une compatibilité HLA entre donneur et receveur, en cas de greffe, comme spécifié ci-dessus et dans un but d'identification des individus (criminologie et recherche de paternité notamment).

En conséquence, la Demanderesse s'est donné pour but de pourvoir à un procédé d'identification rapide et fiable d'allèles, qui a l'avantage de permettre l'identification de la carte allèlique complète d'un sujet, et ce sans nécessiter l'utilisation d'un nombre élevé de sondes oligonucléotidiques (difficulté de réalisation et coût élevé desdites sondes).

La présente invention a pour objet un procédé pour la sélection, à partir d'un ensemble de séquences allèliques d'un gène polymorphe, d'au moins un crible de mutations destiné à spécifier au moins une sonde nucléotidique apte à être utilisée pour la discrimination de tous les allèles, caractérisé en ce qu'il comprend les étapes suivantes :

(a) la sélection de tout ou partie d'une séquence *consensu*s connue dudit gène polymorphe ;

(b) la création d'une matrice des mutations des séquences correspondantes d'allèles connus ;

(c) l'identification des séquences indiscernables par comparaison deux à deux (allèles ayant le même profil de mutations dans la séquence sélectionnée en (a)) et l'exclusion d'un des membres desdits couples ;

(e) l'identification et le dénombrement des mutations obligatoires ou mutations dites marqueurs d'allèles, c'est-à-dire celles qui sont nécessaires et suffisantes pour la distinction de deux allèles par ailleurs identiques (ensemble O des mutations obligatoires) ; et

(f) l'obtention dudit/desdits cribles minimaux de mutations, comprenant au moins les mutations obligatoires de l'étape (e).

Selon un mode de mise en oeuvre avantageux dudit procédé, préalablement à l'étape (e) d'identification et de dénombrement des mutations obligatoires, ledit procédé comprend :

(d) l'identification des mutations similaires dans chacune desdites séquences d'allèles de l'étape (b), de manière à ne traiter dans les étapes suivantes que les mutations non redondantes et constituant l'ensemble U des mutations utiles ; laquelle étape (d) est suivie des étapes (e) et (f) modifiées comme suit :

(e) l'identification et le dénombrement des mutations obligatoires ou mutations dites marqueurs d'allèles, parmi les mutations utiles de l'ensemble U, c'est-à-dire celles qui sont nécessaires et suffisantes pour la distinction de deux allèles par ailleurs identiques (ensemble O' des mutations obligatoires) ; et

(f) si les mutations obligatoires de l'étape (e) ne permettent pas l'obtention directe de cribles de mutations aptes à la différenciation univoque de tous les allèles, on procède à la sélection d'un nombre minimal de mutations utiles de l'étape (d) (sous-ensemble U₁ issu de l'ensemble U des mutations utiles) qui, associées aux mutations obligatoires de l'étape (e), forment le/les cribles de mutations aptes à la différenciation univoque de tous les allèles.

Selon une disposition avantageuse de ce mode de mise en oeuvre, préalablement à l'étape (f), ledit procédé comprend une étape (x) de sélection de mutations utiles de l'étape (d) (sous-ensemble U₂ issu de l'ensemble U des mutations utiles), pour former un groupe des mutations utiles les plus adaptées à la réalisation de sondes oligonucléotidiques aptes à être utilisées pour la différenciation de tous les allèles ; laquelle étape (x) est suivie de l'étape (f) modifiée comme suit :

(f) si les mutations obligatoires ne permettent pas la sélection directe de cribles de mutations aptes à la différenciation univoque de tous les allèles, on procède à la sélection d'un nombre minimal de mutations utiles de l'étape (x) qui, associées aux mutations obligatoires de l'étape (e), forment le/les cribles de mutations aptes à la différenciation univoque de tous les allèles.

De tels cribles de mutations sont particulièrement intéressants pour la sélection et la réalisation d'un nombre restreint de sondes oligonucléotidiques, aptes à être utilisées pour la différenciation de tous les allèles d'un gène polymorphe.

La présente invention a également pour objet un procédé pour l'identification d'allèles (ou séquences allèliques) d'un gène polymorphe, caractérisé en ce qu'il comprend les étapes suivantes :
I - la sélection d'au moins un crible de mutations réalisé à partir d'un ensemble de séquences allèliques d'un gène polymorphe au cours des étapes :
   . (a) à (f) du procédé de sélection d'au moins un crible de mutations tel que défini ci-dessus (y compris les différentes variantes) ; puis
   . (g) le choix, parmi les cribles sélectionnés à l'étape (f) dudit procédé de sélection de cribles de mutations, du crible de mutations le plus adapté à la sélection et à la réalisation de sondes oligonucléotidiques aptes à être utilisées pour la différenciation de tous les allèles ;
II - typage proprement dit d'un allèle X à identifier par :
   (h) une hybridation appropriée dudit allèle X avec les sondes oligonucléotidiques sélectionnées à partir du/des cribles de mutations établis au cours des étapes (a) à (g) ; et
   (i) identification de l'allèle X par détection dudit/desdits hybrides, éventuellement formés au cours de l'étape (h).

De manière avantageuse, lorsque le procédé de sélection de cribles de mutations, comprend l'étape (d) telle que définie ci-dessus, ladite étape (d) a l'avantage d'entraîner une première réduction des mutations à considérer dans la suite des étapes, en éliminant un premier sous-ensemble de mutations (mutations redondantes) et donc de constituer un ensemble U des mutations utiles pour la caractérisation d'un allèle.

Les étapes (e) à (g) ont l'avantage :
. de permettre la sélection d'un sous-ensemble de mutations obligatoires, parmi les mutations utiles de l'ensemble U qui, éventuellement en association avec :
   - soit un sous-ensemble U₁, issu de l'ensemble U des mutations utiles (l'ensemble U₁ correspondant à un nombre minimal de mutations utiles qui, en association avec les mutations obligatoires, forment des cribles de mutations aptes à la différenciation univoque de tous les allèles) ou
   - soit un sous-ensemble U₂, issu de l'ensemble U des mutations utiles et sélectionné pour former un groupe de mutations utiles plus adaptées à la réalisation de sondes oligonucléotidiques convenables, forment des cribles de mutations aptes à la différenciation univoque de tous les allèles ; et

   - de permettre, en raison de la sélection des sondes oligonucléotidiques spécifiques, une identification rapide de l'allèle inconnu.

   En effet, le procédé conforme à l'invention permet outre la sélection d'un nombre restreint de sondes oligonucléotidiques, la sélection de sondes ayant les caractéristiques avantageuses suivantes :
. appariement maximal avec la séquence *consensus* ;
. absence de formation de séquences donnant lieu à la formation d'homo- ou d'hétérodimères non spécifiques ;
. contenu important en bases GC ; et
. absence de séquences répétées polypurines ou polypyrimidines.

De plus, le procédé conforme à l'invention permet l'identification directe des doublets homozygotes et leur différenciation des doublets hétérozygotes.

Dans ce dernier cas, pour établir, *in fine*, le crible de mutations, on met en oeuvre le même procédé que décrit ci-dessus par l'analyse de chaque séquence du doublet à chaque position ; ce sont donc des doublets d'allèles qui sont comparés à tous les autres doublets d'allèles.

Les implications préventives et curatives de la connaissance précise des allèles portés par un sujet donné sont importantes ; le procédé conforme à l'invention permet, dans un temps très court, de résoudre ce problème.

La présente invention a également pour objet l'application du procédé pour la sélection d'au moins un crible de mutations à partir d'un ensemble de séquences allèliques d'un gène polymorphe, à la réalisation d'une banque de données, constituée par l'ensemble des cribles de mutations obtenus par le procédé ci-dessus et destinée à la préparation de sondes oligonucléotidiques aptes à être utilisées pour la discrimination de tous les allèles.

La présente invention a également pour objet des sondes oligonucléotidiques, caractérisées en ce qu'elles sont construites pour la mise en oeuvre d'au moins un crible de mutations issu du procédé de sélection d'au moins un crible de mutations à partir d'un ensemble de séquences allèliques d'un gène polymorphe ou de la banque de données telle que définie ci-dessus, en ce qu'elles comprennent entre 15 et 50 bases et en ce qu'elles sont les plus aptes à s'hybrider à une séquence allèlique pour l'identification d'allèles d'un gène polymorphe.

De telles sondes peuvent éventuellement être marquées à l'aide d'un marqueur tel qu'un isotope radioactif, une enzyme appropriée, un fluorochrome, un anticorps ou un analogue de base ; de telles sondes peuvent également être construites pour être mises en oeuvre dans le procédé de détection et/ou d'identification d'une base nucléotidique spécifique présente sur une séquence d'acide nucléique (mutation) décrit dans la Demande de Brevet européen 412 883, au nom de la Demanderesse.

Selon un mode de réalisation avantageux desdites sondes, elles comprennent une séquence issue de la séquence *consensus* sélectionnée et dont la base nucléotidique située à l'extrémité 3' correspond à une base en amont d'une des bases mutantes du crible de mutations sélectionné.

La présente invention a également pour objet un kit pour l'identification d'allèles d'un gène polymorphe, caractérisé en ce qu'il comprend au moins :
- des quantités appropriées d'une collection de sondes oligonucléotidiques conformes à l'invention ; éventuellement associées à :
- des quantités appropriées d'un réactif de détection des hybrides sonde-séquence à identifier éventuellement formés ; et/ou à
- un tableau d'interprétation du résultat des hybridations obtenues, en fonction du crible de mutations sélectionné.

Selon un mode de réalisation avantageux dudit kit, lesdites sondes comprennent une séquence issue de la séquence *consensus* sélectionnée et dont la base nucléotidique située à l'extrémité 3' correspond à une base en amont d'une des bases mutantes du crible de mutations sélectionné.

Selon un autre mode de réalisation avantageux dudit kit, il comprend en outre :
- des quantités appropriées de quatre bases nucléotidiques modifiées, de manière à être incorporables dans le produit d'extension desdites sondes utilisées comme amorces, tout en bloquant l'élongation dudit produit d'extension.

Un tel mode de réalisation permet la mise en oeuvre du procédé décrit dans la Demande de brevet européen 412 883 au nom de la Demanderesse.

La présente invention a, en outre, pour objet un dispositif pour la mise en oeuvre du procédé conforme à l'invention, caractérisé en ce qu'il comprend au moins :
- des moyens d'entrée de données,
- des moyens de calcul programmés pour générer le/les cribles de mutations,
- des moyens de mémorisation desdits cribles, et
- des moyens aptes à permettre l'identification des allèles à partir des cribles mémorisés.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'au dessin annexé, dans lequel :

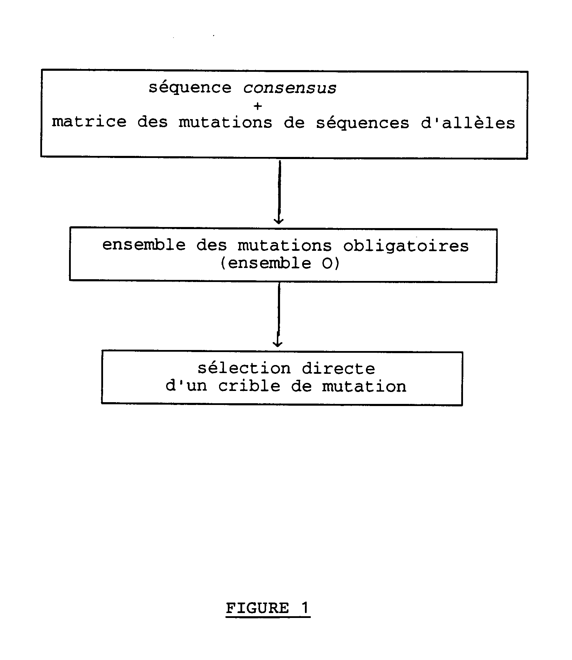

- la figure 1 illustre un mode de réalisation du procédé de sélection d'un crible de mutations dans lequel ledit crible est directement obtenu à partir de l'ensemble O de mutations obligatoires ;

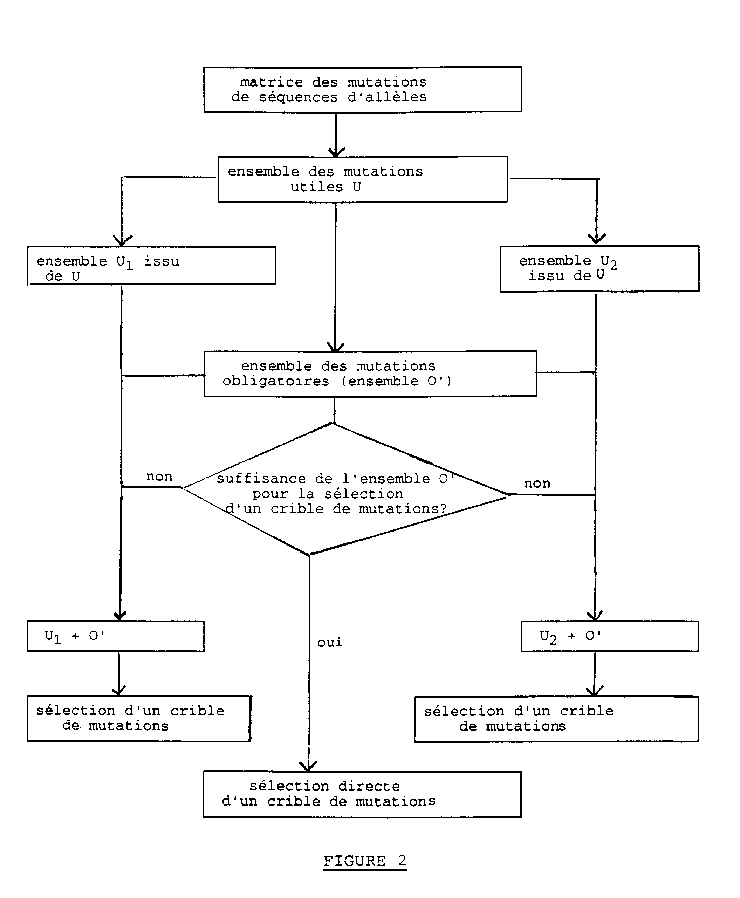

- la figure 2 illustre un autre mode de réalisation dans lequel ledit crible est obtenu à partir d'un ensemble O' de mutations obligatoires issu d'un ensemble U de mutations utiles, lequel ensemble O' est éventuellement associé à un sous-ensemble U₁ ou à un sous-ensemble U₂ de mutations utiles, tels que définis ci-dessus ;

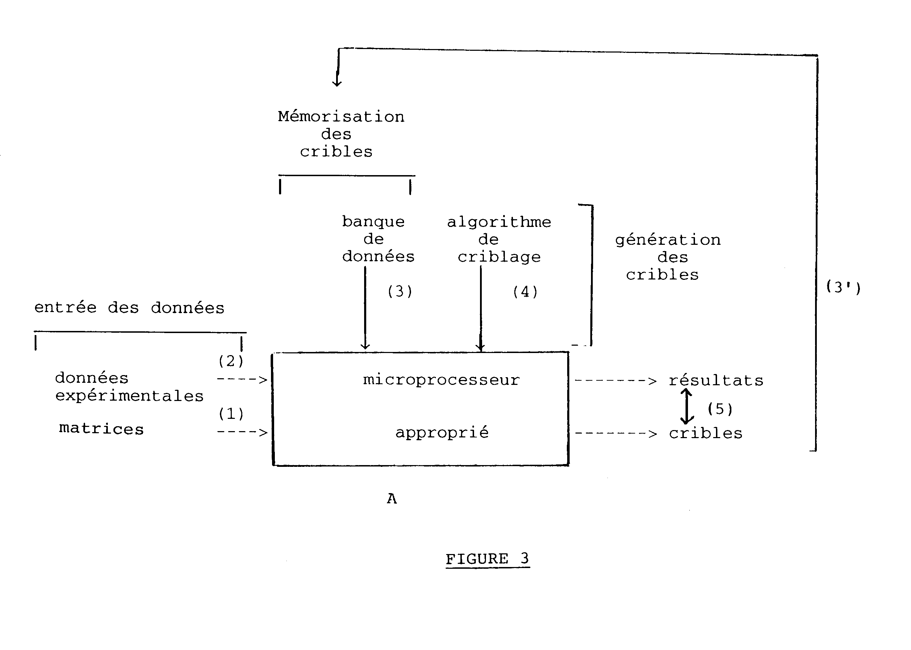

- la figure 3 illustre un dispositif de mise en oeuvre des procédés conformes à l'invention (phase de création et phase d'exploitation) ;
- la figure 4 illustre une matrice de mutations d'une séquence à 7 allèles, dénommée All ;
- la figure 5 illustre l'ensemble U (mutations utiles) en vue d'identifier un couple d'allèles homozygotes de la séquence All ;
- la figure 6 illustre les cribles de mutations aptes à l'identification univoque de tous les couples d'allèles homozygotes de All ;
- la figure 7 illustre l'ensemble U (mutations utiles) en vue d'identifier un couple d'allèles hétérozygotes du gène All ;
- la figure 8 illustre le crible de mutations apte à l'identification univoque de tous les doublets d'allèles hétérozygotes de All ;
- la figure 9 illustre l'ensemble U (mutations utiles) en vue d'identifier un couple d'allèles homozygotes du gène DQβ1 ; et
- la figure 10 illustre l'ensemble des cribles de mutations aptes à l'identification de tous les couples d'allèles homozygotes du gène DQβ1.

Il doit être bien entendu, toutefois, que ces exemples sont donnés uniquement à titre d'illustration de l'objet de l'invention, dont ils ne constituent en aucune manière une limitation.

Un dispositif conforme à l'invention permet la mise en oeuvre des procédés de sélection et d'identification tels que définis ci-dessus aussi bien en phase de création (constitution des cribles) qu'en phase d'exploitation (identification d'un allèle).

En phase de création, la matrice des mutations d'allèles est introduite en (1) dans un microprocesseur (A) approprié et génèrent en (4) au moyen du procédé de sélection d'au moins un crible de mutations conforme à l'invention, un ensemble de cribles, mémorisés en (3, 3') dans une banque de données .

En phase d'exploitation, une séquence à identifier est hybridée avec une collection de sondes convenables, construites pour la mise en oeuvre d'au moins un crible de mutations ; à partir des hybrides obtenus, on identifie la séquence (données expérimentales introduites en (2)) ; on compare le résultat obtenu avec le crible en (5), ce qui permet de préciser de quel allèle il s'agit.

### EXEMPLE 1 : Constitution de cribles de mutations des allèles homozygotes du gène All.

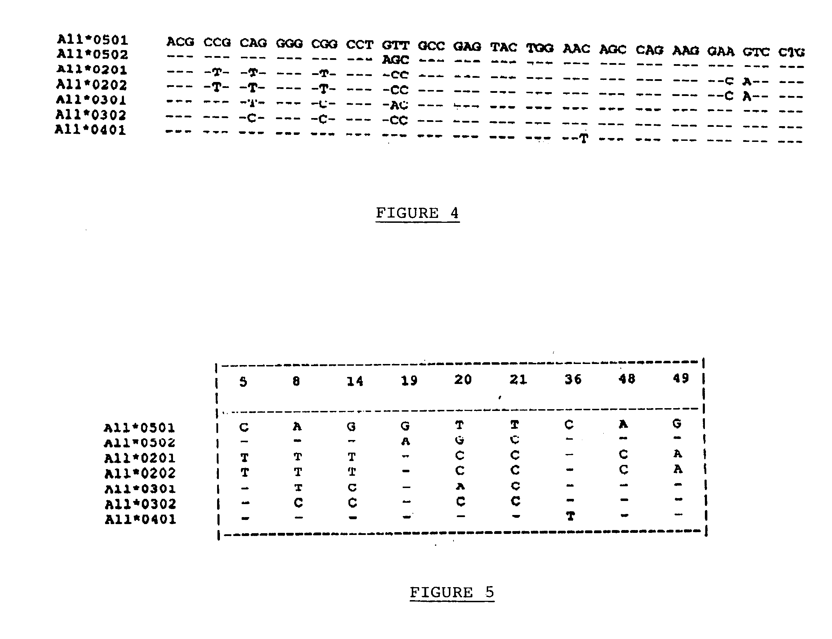

. on sélectionne la séquence All*0501 comme séquence *consensus* comme visible sur la figure 4, dans laquelle la première séquence est considérée comme la séquence *consensus* ; dans les autres séquences seules sont indiquées les mutations par rapport à ladite séquence consensus ;
. on compare les allèles deux à deux et on identifie les mutations utiles pour différencier chaque couple d'allèles : les mutations utiles trouvées avec le procédé conforme à l'invention sont au nombre de 9 :
   5, 8, 14, 19, 20, 21, 36, 48, 49
   conformément à la figure 5.
   Dans cet exemple, un couple d'allèles est indiscernable (couple All*0201 et All*0202) ; l'allèle All*0202 est en conséquence supprimé pour le reste de l'analyse.
. on procède ensuite à la recherche des mutations obligatoires : All*0401 et All*0501 ne diffèrent que par 36.
   Il ressort de cette recherche qu'il n'existe qu'une seule position obligatoire parmi les mutations utiles, il s'agit de la position 36.
. dans le cas présent, la seule mutation obligatoire ne permet pas de discriminer tous les couples d'allèles possibles ; il n'existe pas de "solutions", i.e. de cribles permettant l'identification univoque de tous les allèles considérés, avec un nombre de mutations inférieur à 3 (soit 2 mutations supplémentaires). Tous les cribles de mutations possibles à 3 mutations sont :

1) 36, 5, 20
2) 36, 8, 20
3) 36, 14, 20,

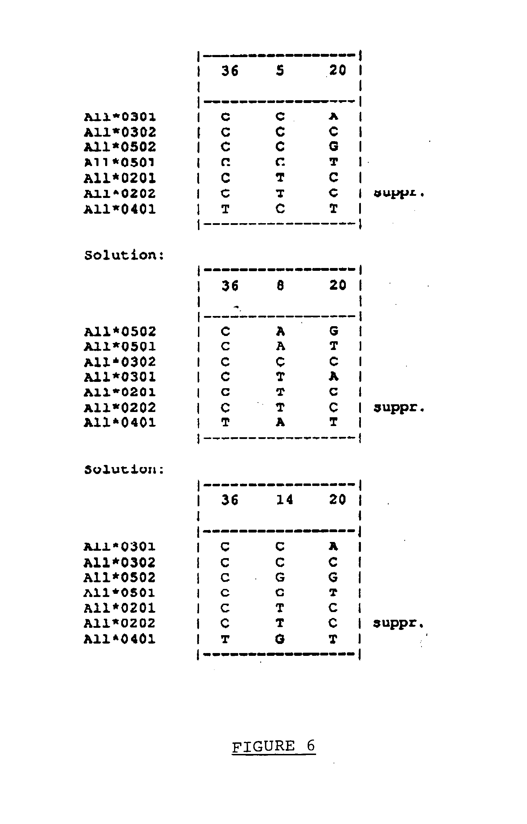

conformément aux figures 6.1 à 6.3 et montrent qu'il est possible d'identifier un allèle du gène All à l'aide de l'un quelconque de ces cribles de mutations.

### EXEMPLE 2 : Constitution de cribles de mutations des allèles hétérozygotes du gène All.

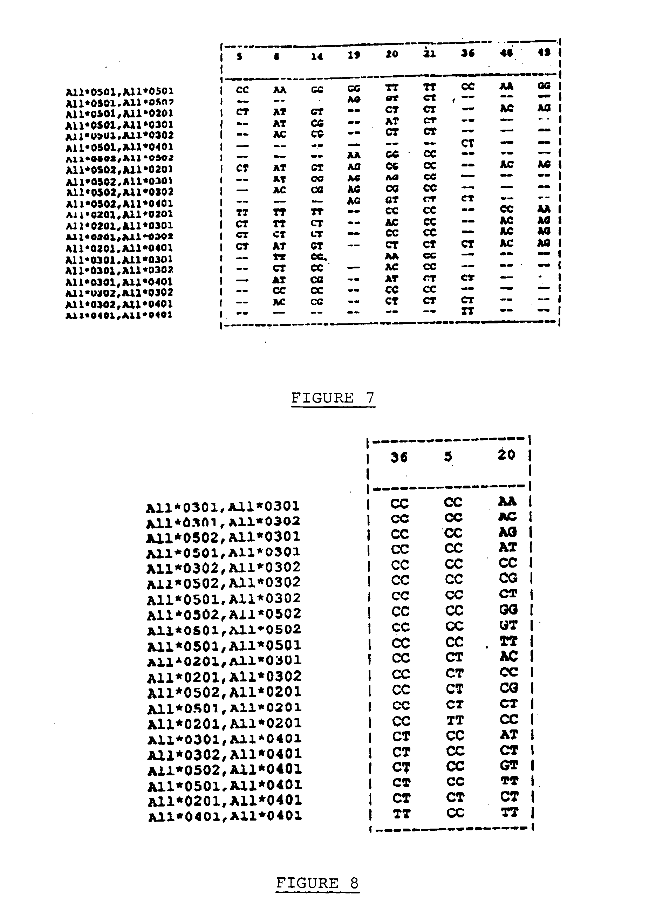

Dans cet exemple, après l'exécution des étapes telles que décrites à l'exemple 1, et qui aboutissent à l'identification des mutations utiles comme visible à la figure 7, on procède à la recherche des mutations obligatoires qui permettent la discrimination de tous les doublets d'allèles :
All*0401, All*0401 et All*0501, All*0401 ne diffèrent que par 36 ;
All*0401, All*0401 et All*0501, All*0501 ne diffèrent que par 36 ;
All*0302, All*0401 et All*0501, All*0302 ne diffèrent que par 36 ;
All*0301, All*0401 et All*0501, All*0301 ne diffèrent que par 36 ;
All*0201, All*0401 et All*0501, All*0201 ne diffèrent que par 36 ;
All*0502, All*0401 et All*0501, All*0502 ne diffèrent que par 36 ;
All*0501, All*0401 et All*0501, All*0501 ne diffèrent que par 36.

Il ressort de cette recherche qu'il n'existe une seule position obligatoire parmi les mutations utiles ; il s'agit de la position 36. Dans cet exemple, cette seule mutation obligatoire ne permet pas de discriminer tous les doublets d'allèles. Il n'existe pas de "solutions" avec un nombre inférieur à 3, soit 2 positions supplémentaires. Un des cribles de mutations qui permet la discrimination de tous les doublets d'allèles est : 36, 5, 20, conformément à la figure 8.

### EXEMPLE 3 : Typage d'un individu hétérozygote All.

Le crible de mutations de l'exemple 2 est choisi pour identifier des allèles, car il est le plus adapté à la réalisation de sondes qui répondent aux critères de sélection définis ci-dessus (appariement maximal avec la séquence *consensus*, absence de séquences donnant lieu à la formation d'homo- ou d'hétérodimères, contenu important en bases GC et absence de séquences répétées polypurines ou polypyrimidine).

Des sondes de 20 oligonucléotides sont synthétisées de manière à ce que la position 3' desdites sondes correspondent à une base située juste en amont d'une des positions des cribles ci-dessus, de manière à ce que lorsque l'on procède à l'hybridation et à l'extension dans les conditions de la Demande de Brevet européen précitée, on puisse vérifier laquelle/lesquelles des bases s'apparie(nt). L'utilisation d'un tel panel de sondes permet d'identifier
. chez l'individu 1, la séquence CC CT CC qui, en référence au crible choisi permet d'identifier le couple d'allèles All*0201, All*0302, et
. chez l'individu 2 testé, la séquence CC CT CG qui, en référence au crible choisi permet d'identifier le couple d'allèles All*0502, All*0201.

### EXEMPLE 4 : Constitution de cribles de mutations des allèles homozygotes du gène HLA-DQβ1

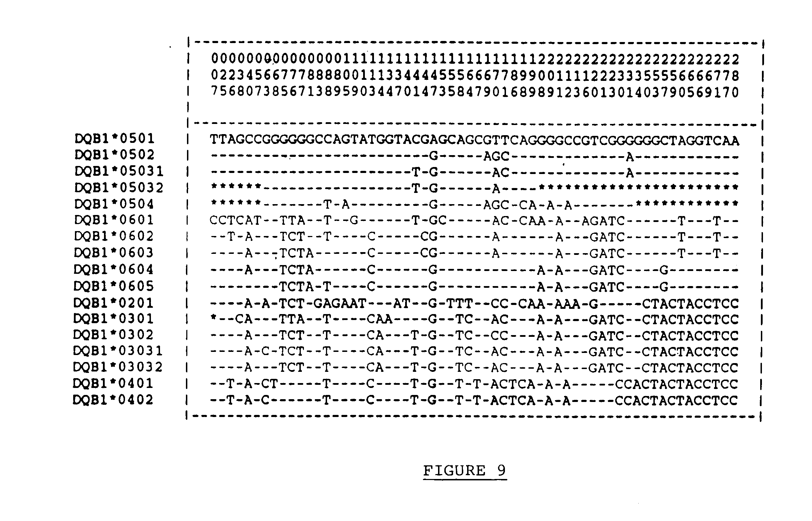

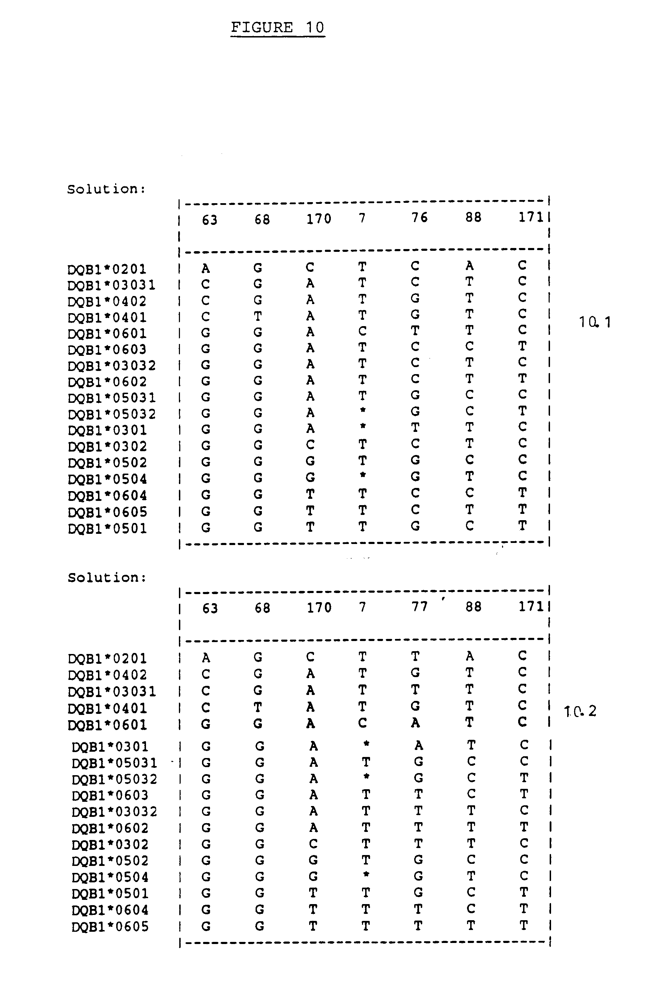

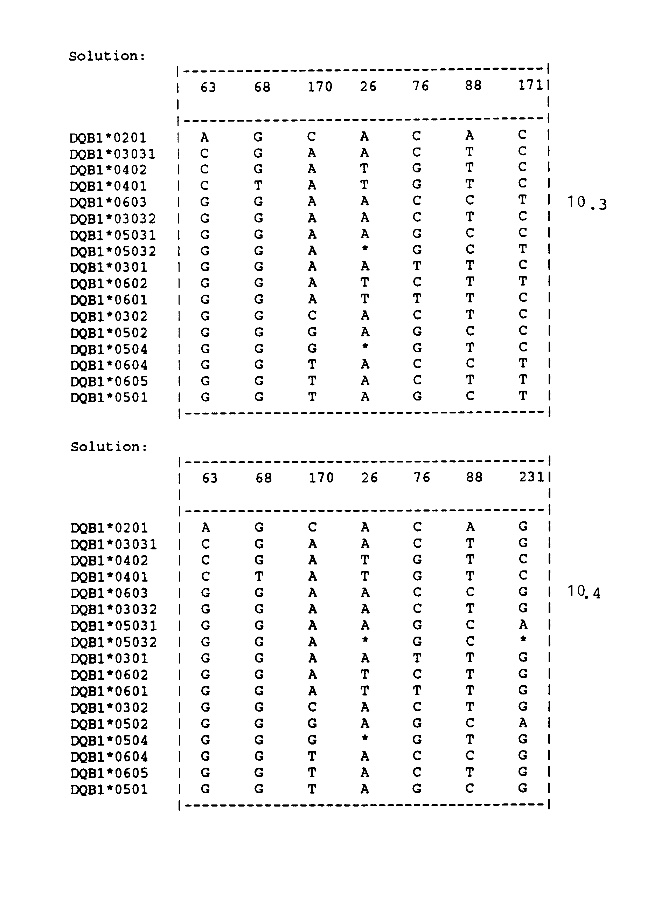

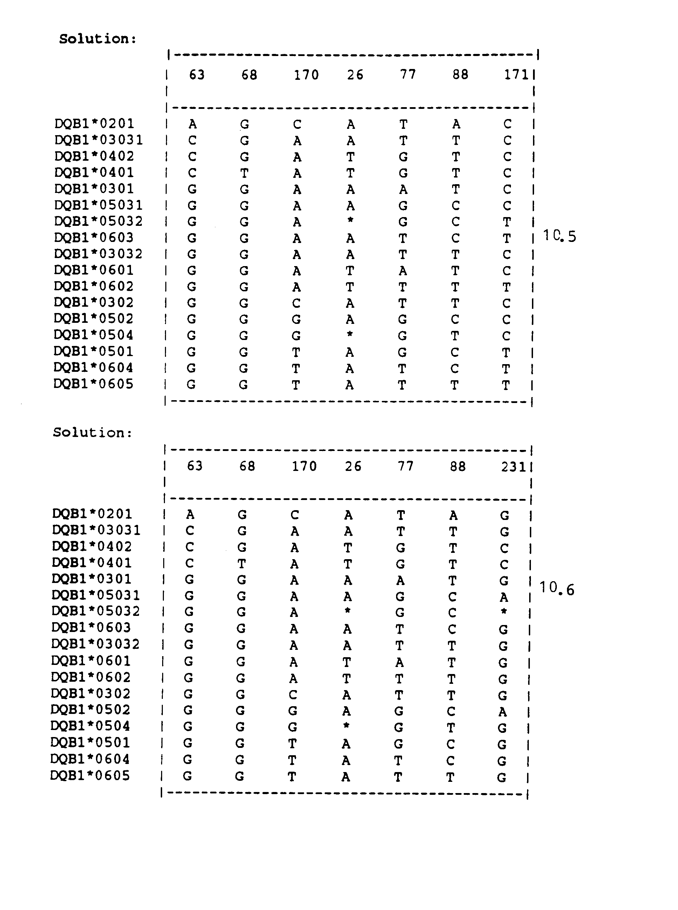

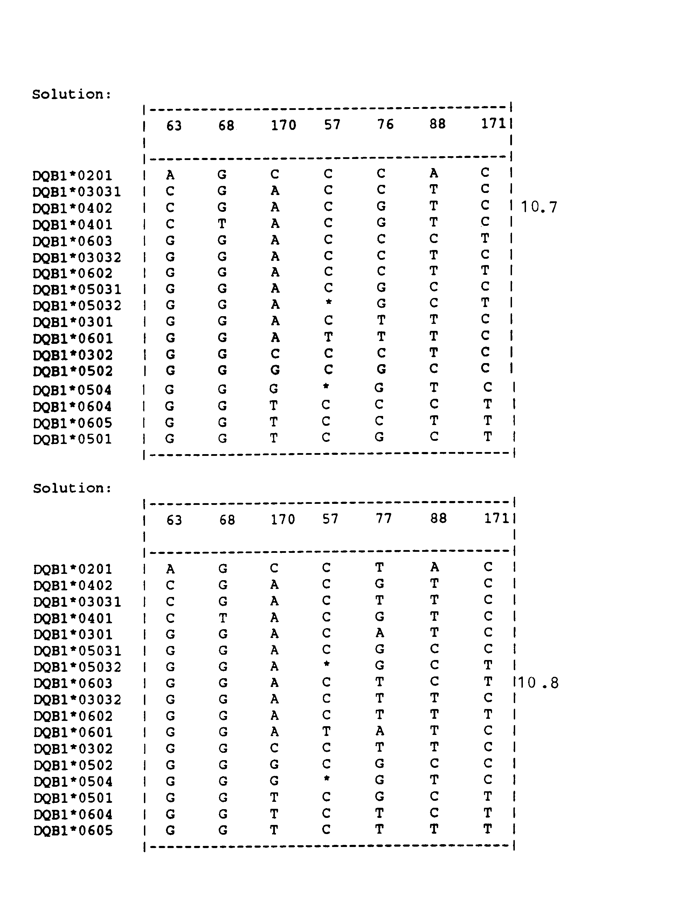

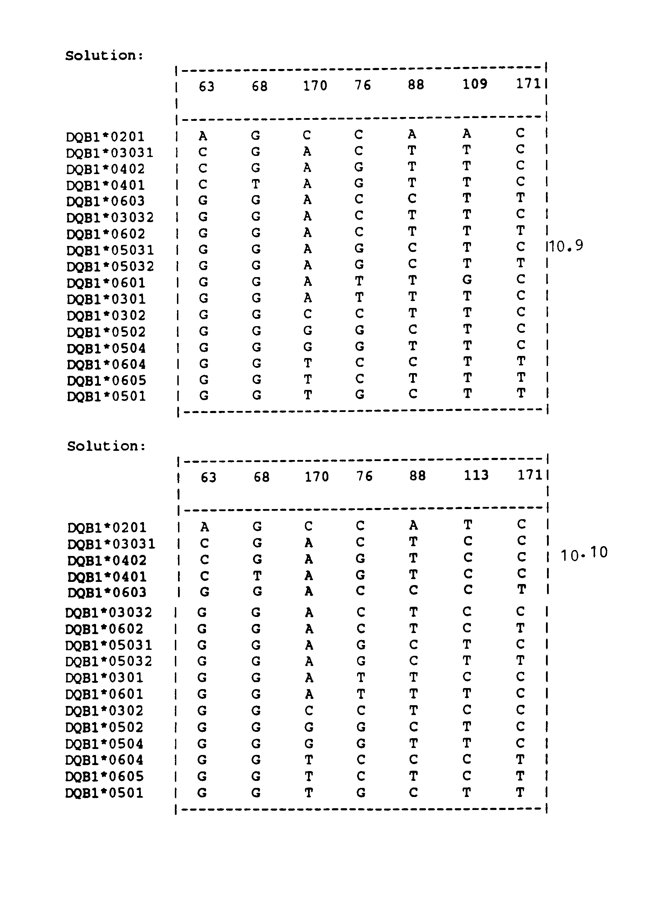

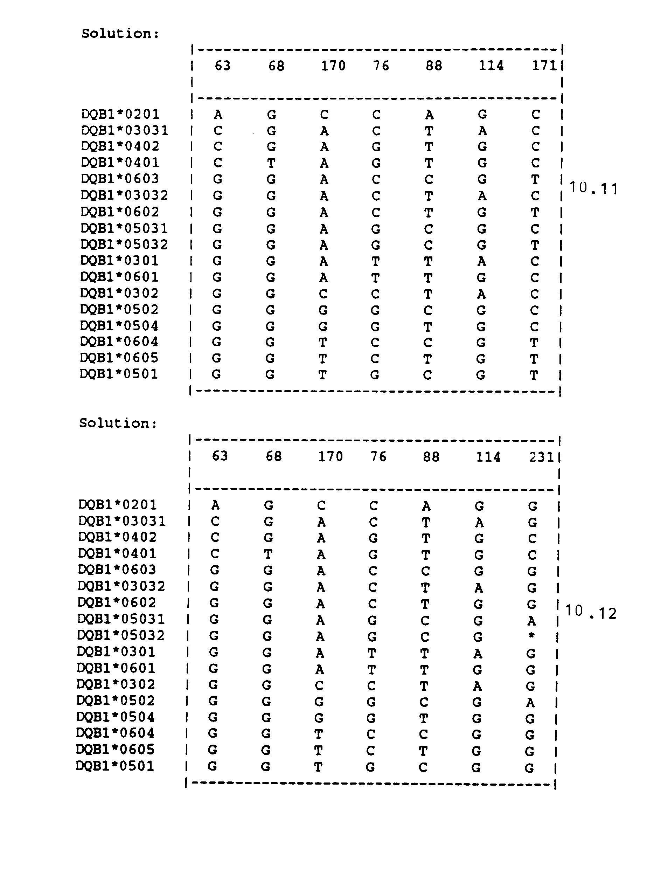

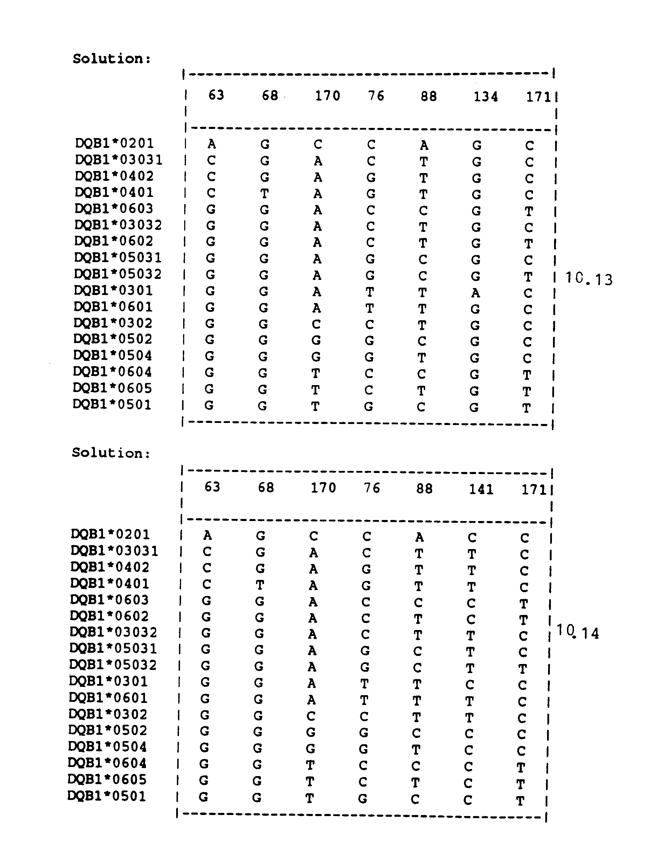

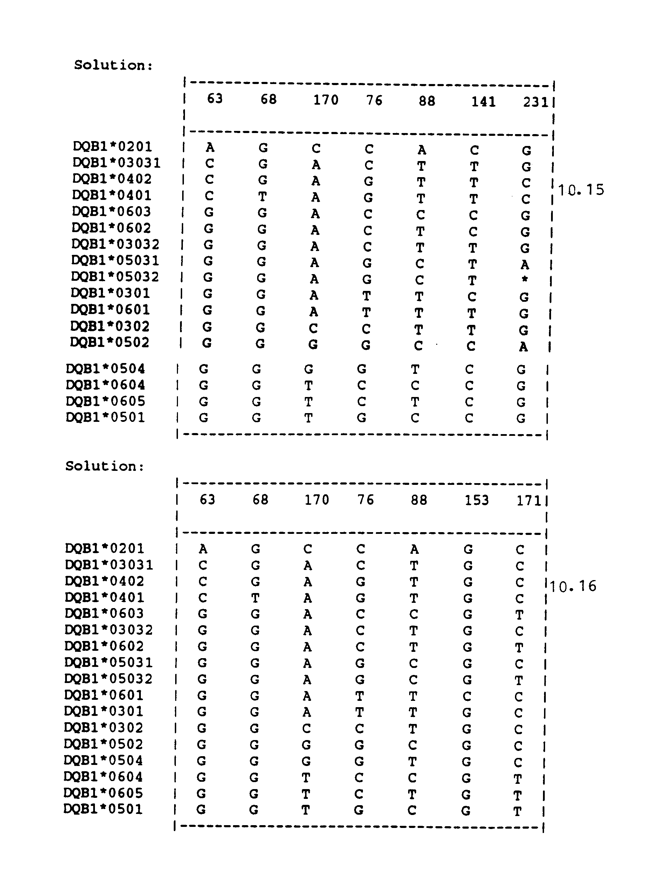

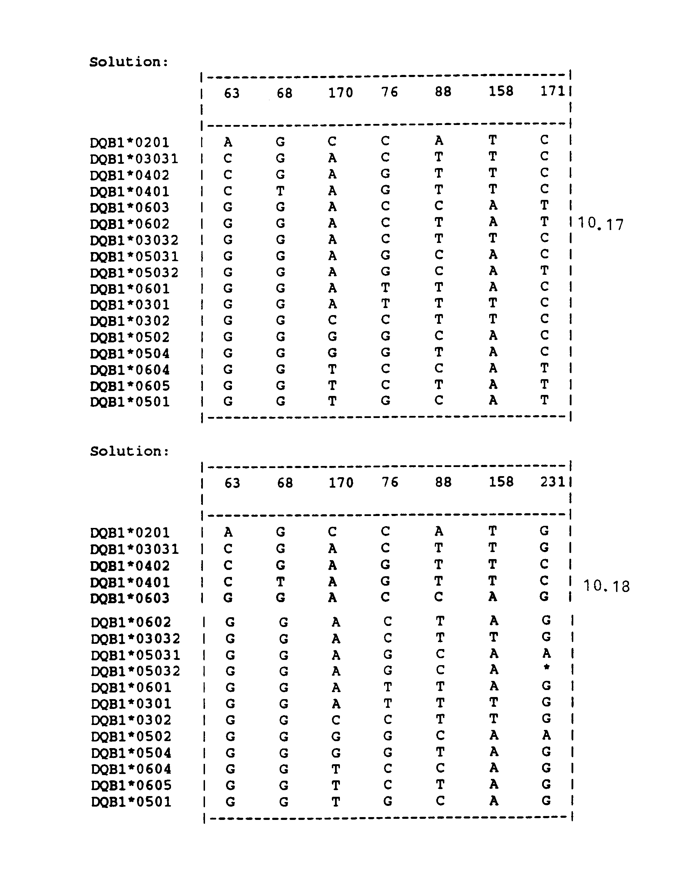

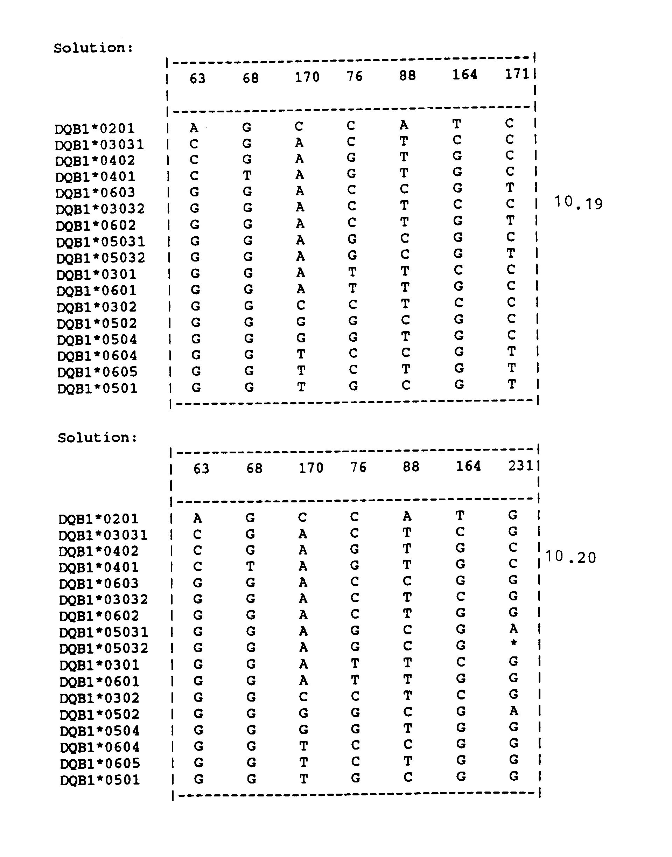

La nomenclature des facteurs du système HLA a été publiée en 1990 dans Immunogenetics, 31, 131-140 et l'exemple qui suit illustre la constitution d'un crible de mutations des allèles du gène HLA-DQβ1, tels que définis dans cet article.
. on sélectionne la séquence DQβ1*0501 (position 1 à position 300) comme séquence *consensus* ;
. on identifie les positions des mutations similaires, afin de ne les considérer qu'une fois ; on obtient le résultat suivant :
   * la mutation 25 est similaire à la 7 ;
   * la mutation 140 est similaire à la 110 ;
   * la mutation 186 est similaire à la 167 ;
   * la mutation 266 est similaire à la 250 ;
   * la mutation 269 est similaire à la 259 ;
   * la mutation 280 est similaire à la 277 ;
   en conséquence, les mutations 25, 140, 186, 266, 269 et 280 sont ignorées dans l'étape suivante du procédé (les numéros correspondent aux positions des mutations sur la séquence).
. on compare les allèles deux à deux et on identifie les mutations utiles pour différencier chaque couple d'allèles : les mutations utiles trouvées avec le procédé conforme à l'invention sont au nombre de 54 :
   7 26 38 40 57 63 68 75 76 77 81 83 88 89 105 109 110 113 114 134 137 141 144 147 153 155 158 164 167 169 170 171 198 199 208 209 211 212 213 216 220 221 223 230 231 234 250 253 257 259 260 265 271 277, conformément à la figure 9.
   Dans cet exemple, tous les couples d'allèles peuvent être différenciés.
. on procède ensuite à la recherche des mutations obligatoires :
   DQβ1*0402 et DQβ1*0401 ne diffèrent que par 68,
   DQβ1*03032 et DQβ1*03031 ne diffèrent que par 63,
   DQβ1*03032 et DQβ1*0302 ne diffèrent que par 170.
   Il ressort de cette recherche que les positions obligatoires parmi les mutations utiles sont 63, 68 et 170.
. dans le cas présent, les 3 mutations obligatoires ne permettent pas de discriminer tous les couples d'allèles possibles ; il n'existe pas de solutions avec un nombre de mutations inférieur à 7 (soit 4 mutations supplémentaires. Tous les cribles de mutations possibles à 7 mutations sont :
   1- 63, 68, 170, 7, 76, 88, 171,
   2- 63, 68, 170, 7, 77, 88, 171,
   3- 63, 68, 170, 26, 76, 88, 171,
   4- 63, 68, 170, 26, 76, 88, 231,
   5- 63, 68, 170, 26, 77, 88, 171,
   6- 63, 68, 170, 26, 77, 88, 231,
   7- 63, 68, 170, 57, 76, 88, 171,
   8- 63, 68, 170, 57, 77, 88, 171,
   9- 63, 68, 170, 76, 88, 109, 171,
   10- 63, 68, 170, 76, 88, 113, 171,
   11- 63, 68, 170, 76, 88, 114, 171,
   12- 63, 68, 170, 76, 88, 114, 231,
   13- 63, 68, 170, 76, 88, 134, 171,
   14- 63, 68, 170, 76, 88, 141, 171,
   15- 63, 68, 170, 76, 88, 141, 231,
   16- 63, 68, 170, 76, 88, 153, 171,
   17- 63, 68, 170, 76, 88, 158, 171,
   18- 63, 68, 170, 76, 88, 158, 231,
   19- 63, 68, 170, 76, 88, 164, 171, et
   20- 63, 68, 170, 76, 88, 164, 231,
   conformément aux figures 10.1 à 10.20 (dans lesquelles l'allèle DQβ1 est représenté par DQβ1) et montrent qu'il est possible d'identifier un allèle du gène DQβ1 à l'aide de l'un quelconque de ces cribles de mutations.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée de la présente invention.

## Revendications

**1°)** Procédé pour la sélection à partir d'un ensemble de séquences allèliques d'un gène polymorphe, d'au moins un crible de mutations destiné à spécifier au moins une sonde nucléotidique apte à être utilisée pour la discrimination de tous les allèles, caractérisé en ce qu'il comprend les étapes suivantes :
(a) la sélection de tout ou partie d'une séquence *consensus* connue dudit gène polymorphe ;
(b) la création d'une matrice des mutations des séquences correspondantes d'allèles connus ;
(c) l'identification des séquences indiscernables par comparaison deux à deux (allèles ayant le même profil de mutations dans la séquence sélectionnée en (a)) et l'exclusion d'un des membres desdits couples ;
(e) l'identification et le dénombrement des mutations obligatoires ou mutations dites marqueurs d'allèles, c'est-à-dire celles qui sont nécessaires et suffisantes pour la distinction de deux allèles par ailleurs identiques (ensemble O des mutations obligatoires) ; et
(f) l'obtention dudit/desdits cribles minimaux de mutations, comprenant au moins les mutations obligatoires de l'étape (e).

**2°)** Procédé de sélection selon la revendication 1, caractérisé en ce que, préalablement à l'étape (e) d'identification et de dénombrement des mutations obligatoires, ledit procédé comprend :
(d) l'identification des mutations similaires dans chacune desdites séquences d'allèles de l'étape (b), de manière à ne traiter dans les étapes suivantes que les mutations non redondantes et constituant l'ensemble U des mutations utiles ; laquelle étape (d) suivie des étapes (e) et (f) modifiées comme suit :
(e) l'identification et le dénombrement des mutations obligatoires ou mutations dites marqueurs d'allèles, parmi les mutations utiles de l'ensemble U, c'est-à-dire celles qui sont nécessaires et suffisantes pour la distinction de deux allèles par ailleurs identiques (ensemble O' des mutations obligatoires) ; et
(f) si les mutations obligatoires de l'étape (e) ne permettent pas l'obtention directe de cribles de mutations aptes à la différenciation univoque de tous les allèles, on procède à la sélection d'un nombre minimal de mutations utiles de l'étape (d) (sous-ensemble U₁ issu de l'ensemble U des mutations utiles) qui, associées aux mutations obligatoires de l'étape (e), forment le/les cribles de mutations aptes à la différenciation univoque de tous les allèles.

**3°)** Procédé de sélection selon la revendication 2, caractérisé en ce que, préalablement à l'étape (f), ledit procédé comprend une étape (x) de sélection de mutations utiles de l'étape (d) (sous-ensemble U₂ issu de l'ensemble U des mutations utiles), pour former un groupe des mutations utiles les plus adaptées à la réalisation de sondes oligonucléotidiques aptes à être utilisées pour la différenciation de tous les allèles ; laquelle étape (x) est suivie de l'étape (f) modifiée comme suit :
(f) si les mutations obligatoires ne permettent pas la sélection directe de cribles de mutations aptes à la différenciation univoque de tous les allèles, on procède à la sélection d'un nombre minimal de mutations utiles de l'étape (x) qui, associées aux mutations obligatoires de l'étape (e), forment le/les cribles de mutations aptes à la différenciation univoque de tous les allèles.

**4°)** Procédé pour l'identification d'allèles d'un gène polymorphe, caractérisé en ce qu'il comprend les étapes suivantes :
I - la sélection d'au moins un crible de mutations réalisé à partir d'un ensemble de séquences allèliques d'un gène polymorphe au cours des étapes :
. (a) à (f) du procédé de sélection selon l'une quelconque des revendications 1 à 3 ; puis
. (g) le choix, parmi les cribles sélectionnés à l'étape (f) dudit procédé de sélection de cribles de mutations, du crible de mutations le plus adapté à la réalisation de sondes oligonucléotidiques aptes à être utilisées pour la différenciation de tous les allèles ;
II - typage proprement dit d'un allèle X à identifier par :
(h) une hybridation appropriée dudit allèle X avec les sondes oligonucléotidiques sélectionnées à partir du/des cribles de mutations établis au cours des étapes (a) à (g) ; et
(i) identification de l'allèle X par détection dudit/desdits hybrides, éventuellement formés au cours de l'étape (h).
**5°)** Application du procédé selon l'une quelconque des revendications 1 à 3, à la réalisation d'une banque de données constituée par l'ensemble des cribles de mutations obtenus par ledit procédé et destinée à la préparation de sondes nucléotidiques aptes à être utilisées pour la discrimination de tous les allèles.
**6°)** Sondes oligonucléotidiques, caractérisées en ce qu'elles sont construites pour la mise en oeuvre d'au moins un crible de mutations issu du procédé de sélection selon l'une quelconque des revendications 1 à 3 ou de la banque de données réalisée selon la revendication 5, en ce qu'elles comprennent entre 15 et 50 bases et en ce qu'elles sont les plus aptes à s'hybrider à une séquence allèlique pour l'identification d'allèles d'un gène polymorphe.
**7°)** Sondes selon la revendication 6, caractérisées en ce qu'elles comprennent une séquence issue de la séquence *consensus* sélectionnée et dont la base nucléotidique située à l'extrémité 3' correspond à une base en amont d'une des bases mutantes du crible de mutations sélectionné.
**8°)** Kit pour l'identification d'allèles d'un gène polymorphe, caractérisé en ce qu'il comprend au moins :
- des quantités appropriées d'une collection de sondes oligonucléotidiques selon la revendication 6 ou la revendication 7 ; éventuellement associées à :
- des quantités appropriées d'un réactif de détection des hybrides sonde-séquence à identifier éventuellement formés ; et/ou à
- un tableau d'interprétation du résultat des hybridations obtenues, en fonction du crible de mutations sélectionné.
**9°)** Kit selon la revendication 8, caractérisé en ce que lesdites sondes comprennent une séquence issue de la séquence *consensus* sélectionnée et dont la base nucléotidique située à l'extrémité 3' correspond à une base en amont d'une des bases mutantes du crible de mutations sélectionné.
**10°)** Kit selon la revendication 8 ou la revendication 9, caractérisé en ce qu'il comprend en outre :
- des quantités appropriées de quatre bases nucléotidiques modifiées, de manière à être incorporables dans le produit d'extension desdites sondes utilisées comme amorces, tout en bloquant l'élongation dudit produit d'extension.
**11°)** Dispositif pour la mise en oeuvre des procédés selon l'une quelconque des revendications 1 à 4, caractérisé en ce qu'il comprend au moins :
- des moyens d'entrée de données (1, 2),
- des moyens de calcul programmés pour générer le/les cribles de mutations (4),
- des moyens de mémorisation desdits cribles (3, 3'), et
- des moyens (5) aptes à permettre l'identification des allèles à partir des cribles mémorisés.
